(19) Europäisches Patentamt
European Patent Office
Office européen des brevets

(11) EP 4 504 401 B1

# (12) EUROPÄISCHE PATENTSCHRIFT

(45) Veröffentlichungstag und Bekanntmachung des Hinweises auf die Patenterteilung:
**08.04.2026 Patentblatt 2026/15**

(21) Anmeldenummer: **22818692.0**

(22) Anmeldetag: **18.11.2022**

(51) Internationale Patentklassifikation (IPC):
***C07C 29/70*** *(2006.01)* ***C07C 67/03*** *(2006.01)*
***B01J 23/16*** *(2006.01)* ***B01J 23/18*** *(2006.01)*
***C07C 31/30*** *(2006.01)* ***C07C 69/82*** *(2006.01)*
***C08G 63/183*** *(2006.01)* ***C08J 11/18*** *(2006.01)*
***C08J 11/22*** *(2006.01)*

(52) Gemeinsame Patentklassifikation (CPC):
(C-Sets verfügbar)
**B01J 23/18; B01J 23/16; C07C 29/705; C07C 31/30; C07C 67/03; C07C 69/82; C08G 63/183; C08J 11/18; C08J 11/22;**
C08J 2367/02; Y02W 30/62 (Forts.)

(86) Internationale Anmeldenummer:
**PCT/EP2022/082369**

(87) Internationale Veröffentlichungsnummer:
**WO 2023/193941 (12.10.2023 Gazette 2023/41)**

## (54) VERBESSERTES VERFAHREN ZUR DEPOLYMERISIERUNG VON POLYETHYLENTEREPHTHALAT

IMPROVED METHOD FOR THE DEPOLYMERISATION OF POLYETHYLENE TEREPHTHALATE

PROCÉDÉ AMÉLIORÉ DE DÉPOLYMÉRISATION DU POLYÉTHYLÈNE TÉRÉPHTALATE

(84) Benannte Vertragsstaaten:
**AL AT BE BG CH CY CZ DE DK EE ES FI FR GB GR HR HU IE IS IT LI LT LU LV MC ME MK MT NL NO PL PT RO RS SE SI SK SM TR**

(30) Priorität: **04.04.2022 EP 22166568**

(43) Veröffentlichungstag der Anmeldung:
**12.02.2025 Patentblatt 2025/07**

(73) Patentinhaber: **Evonik Operations GmbH**
**45128 Essen (DE)**

(72) Erfinder:
- **REINSBERG, Philip Heinrich**
**53359 Rheinbach (DE)**
- **RUWWE, Johannes**
**63457 Hanau (DE)**
- **WOLF, Jörn Klaus Erich**
**45130 Essen (DE)**
- **GÄRTNER, Felix**
**45721 Haltern am See (DE)**
- **MALTER, Jutta**
**64295 Darmstadt (DE)**
- **ROETTGER, Dirk**
**50672 Köln (DE)**

(74) Vertreter: **Evonik Patent Association**
**c/o Evonik Industries AG**
**IP Management**
**Postcode 84/339**
**Rodenbacher Chaussee 4**
**63457 Hanau (DE)**

(56) Entgegenhaltungen:
**EP-A1- 1 457 479**
**WO-A1-2022/035725**
**DE-C- 513 677**
**FR-A- 1 227 089**
**US-A1- 2019 390 035**

Anmerkung: Innerhalb von neun Monaten nach Bekanntmachung des Hinweises auf die Erteilung des europäischen Patents im Europäischen Patentblatt kann jedermann nach Maßgabe der Ausführungsordnung beim Europäischen Patentamt gegen dieses Patent Einspruch einlegen. Der Einspruch gilt erst als eingelegt, wenn die Einspruchsgebühr entrichtet worden ist. (Art. 99(1) Europäisches Patentübereinkommen).



(Forts. nächste Seite)

- **DUQUE-INGUNZA I. ET AL: "Process optimization for catalytic glycolysis of post-consumer PET wastes", JOURNAL OF CHEMICAL TECHNOLOGY AND BIOTECHNOLOGY, vol. 89, no. 1, 1 January 2014 (2014-01-01), Hoboken, USA, pages 97 - 103, XP055956431, ISSN: 0268-2575, DOI: 10.1002/jctb.4101**
- **WANG SHAOBO ET AL: "Sodium titanium tris(glycolate) as a catalyst for the chemical recycling of poly(ethylene terephthalate) via glycolysis and repolycondensation", POLYMER DEGRADATION AND STABILITY, vol. 114, 1 April 2015 (2015-04-01), GB, pages 105 - 114, XP055966633, ISSN: 0141-3910, DOI: 10.1016/j.polymdegradstab.2015.02.006**
- **WANG S: ""Kinetics of catalytic glycolysis of PET with sodium titanium tris(glycolate)", 1 May 2015 (2015-05-01), XP055972145, Retrieved from the Internet <URL:https://www.proceedings.com/53339.html>**


(52) Gemeinsame Patentklassifikation (CPC): (Forts.)

C-Sets
**C07C 29/705, C07C 31/30;**
**C07C 67/03, C07C 69/82**

## Beschreibung

Die vorliegende Erfindung betrifft ein Verfahren zur Depolymerisierung von Polyethylenterephthalat (= **"PET"),** in welchem **PET** mit Natrium- oder Kaliumethylenglykolat, welches durch eine Reaktivdestillation erhalten wurde, zu einer Mischung $M_1$ umfassend Bis-2-hydroxyethylterephthalat (= **"BHET";** CAS-Nr.: 959-26-2) umgesetzt wird.

Das erfindungsgemäße Verfahren zeichnet sich dadurch aus, dass **BHET** einen besonders hohen Anteil unter den Spaltprodukten in der Mischung $\mathbf{M_1}$ bildet. Dadurch liefert das erfindungsgemäße Verfahren eine hohe Ausbeute an **BHET,** welches direkt für die erneute **PET-Herstellung** eingesetzt werden kann.

Die vorliegende Erfindung betrifft somit auch ein Verfahren zum Recycling von **PET,** in dem das im Verfahren zur Depolymerisierung von **PET** erhaltene **BHET,** gegebenenfalls nach weiterer Reinigung aus $\mathbf{M_1}$**,** wieder zu **PET** polymerisiert wird.

## Hintergrund der Erfindung

Polyethylenterephthalat (= **"PET")** ist einer der bedeutendsten Kunststoffe, der in Textilfasern, als Folien und als Material für Kunststoffflaschen verwendet wird. Allein 2007 lag die in Kunststoffflaschen verwendete Menge bei ~ $10^7$ t (W. Caseri, Polyethylenterephthalate, RD-16-03258 (2009) in F. Böckler, B. Dill, G. Eisenbrand, F. Faupel, B. Fugmann, T. Gamse, R. Matissek, G. Pohnert, A. Rühling, S. Schmidt, G. Sprenger, RÖMPP [Online], Stuttgart, Georg Thieme Verlag, Januar 2022).

Aufgrund seiner Haltbarkeit und die auf **PET** zurückzuführenden Müllmengen stellt es eine der größten ökologischen Herausforderungen der Gegenwart dar. Die Lösung dieses Problems liegt in der Vermeidung und in der effizienten Wiederverwertung von **PET.**

Im Stand der Technik werden mehrere Verfahren zur Spaltung von **PET** vorgeschlagen.

GB 784,248 A beschreibt die Methanolyse von **PET.**

Hydrolytische Verfahren zur Depolymerisierung von **PET** beschreiben JP 2000-309663 A, US 4,355,175 A und T. Yoshioka, N. Okayama, A. Okuwaki, Ind. Eng. Chem. Res. 1998, 37, 336 - 340.

Die Umsetzung von **PET** mit Glykol wird in der EP 0723951 A1, der US 3,222,299 A, der WO 2020/002999 A2, von S.R. Shukla, A.M. Harad, Journal of Applied Polymer Science 2005, 97, 513 - 517 (im Folgenden "Shukla & Harad") und von N.D. Pingale, S.R. Shukla, European Polymer Journal 2008,44, 4151 - 4156 beschrieben. EP1457479A1 offenbart ein Verfahren zur Synthese und Isolierung von BHET aus PET mit NaOH in 1,2-Ethylenglykol. In EP1457479A1 wird die Verwendung einer reaktiven Destillation zur Herstellung eines Glykolytes nicht offenbart.

Shukla & Harad beschreiben, dass bei der PET-Glykolyse Bis-2-hydroxyethyl-terephthalat (= **"BHET")** entsteht. Dieses Spaltprodukt kann gleichzeitig als Edukt zur Herstellung neuen **PET**s eingesetzt werden.

Es besteht demnach ein Interesse an Verfahren zur Depolymerisierung von **PET**, bei dem ein möglichst hoher Anteil an **BHET** unter den Spaltprodukten erhalten wird.

Die Aufgabe der vorliegenden Erfindung bestand darin, ein solches Verfahren zur Verfügung zu stellen.

## Kurzbeschreibung der Erfindung

Es wurde nun überraschend ein Verfahren gefunden, das die erfindungsgemäße Aufgabe löst.

Die vorliegende Erfindung betrifft ein Verfahren zur Depolymerisierung von Polyethylenterephthalat **PET,** umfassend die folgenden Schritte:

(a) $M_AOR$ und Glykol werden in einer Reaktivdestillation umgesetzt, wodurch eine Lösung $\mathbf{S_{AP}}$ umfassend Glykol und $M_A$-Glykolat erhalten wird, wobei $M_A$ ein Alkalimetall ausgewählt aus Natrium, Kalium, bevorzugt $M_A$ = Natrium ist, und wobei R ein Alkylrest mit 1 bis 4, insbesondere 1 bis 3, bevorzugt 1 oder 2 Kohlenstoffatomen ist, und am bevorzugtesten R = Methyl ist,

(b) Umsetzung der Lösung $\mathbf{S_{AP}}$ mit **PET** zu einer Mischung $\mathbf{M_1}$ umfassend Bis-2-hydroxyethylterephthalat **(BHET).**

Bevorzugt wird $\mathbf{S_{AP}}$ im Schritt (a) dadurch erhalten wird, dass ein Eduktstrom $\mathbf{S_{AE1}}$ umfassend Glykol mit einem Eduktstrom $\mathbf{S_{AE2}}$ umfassend $M_AOR$ im Gegenstrom in einer Reaktivrektifikationskolonne $\mathbf{RR_A}$ zu einem Rohprodukt $\mathbf{RP_A}$ umfassend $M_A$-Glykolat, ROH, Glykol, $M_AOR$ umgesetzt wird,
wobei $\mathbf{S_{AP}}$ am unteren Ende von $\mathbf{RR_A}$ als Sumpfproduktstrom entnommen wird.

Optional wird am oberen Ende von $\mathbf{RR_A}$ ein Brüdenstrom $\mathbf{S_{AB}}$ umfassend ROH und gegebenenfalls Glykol entnommen.

In einem weiteren Aspekt betrifft die vorliegende Erfindung ein Verfahren zum Recycling von **PET,** in welchem in einem Schritt (ζ) das im erfindungsgemäßen Verfahren zur Depolymerisierung erhaltene **BHET** zu **PET** polymerisiert

wird.

Es wurde überraschend gefunden, dass bei Umsetzung des **PETs** mit dem durch Reaktivdestillation aus dem Alkoholat $M_AOR$ erhaltenen $\mathbf{S_{AP}}$ ein höherer Anteil an **BHET** erhalten wird als in herkömmlichen Verfahren, in denen die alkalische Alkalimetall-Glykolatlösung durch Mischung des Glykols im entsprechenden Alkalimetallhydroxid erhalten wird.

## Detaillierte Beschreibung der Erfindung

Es wurde nun überraschend gefunden, dass die Glykolyse von **PET** besonders effizient verläuft, wenn Natrium- oder Kaliumglykolat eingesetzt wird, welches durch Reaktivdestillation erhalten wurde. Bei der erfindungsgemäßen Reaktivdestillation wird das Glykolat durch Umsetzung des entsprechenden Alkalimetallalkoholats $M_AOR$ mit Glykol gewonnen. Es wurde nun beobachtet, dass im erfindungsgemäßen Verfahren im Vergleich zu den Verfahren des Standes der Technik, in denen Glykolat eingesetzt wird, welches durch Auflösen der Alkalimetallhydroxide in Glykol erhalten wurde, ein höherer Anteil von **BHET** im Spaltprodukt erhalten wird.

### 1. Schritt (a): Reaktivdestillation zum Erhalt der Lösung $\mathbf{S_{AP}}$ umfassend Glykol und $M_A$-Glykolat

Die im erfindungsgemäßen Verfahren eingesetzte Lösung $\mathbf{S_{AP}}$ umfassend Glykol und $M_A$-Glykolat wird erfindungsgemäß mittels Reaktivdestillation durch Umsetzung von $M_AOR$ und Glykol erhalten.

$M_A$ ist ein Alkalimetall ausgewählt aus Natrium, Kalium. $M_A$ ist bevorzugt Natrium.

R ist ein Alkylrest mit 1 bis 4 Kohlenstoffatomen, bevorzugt ein Alkylrest mit 1 bis 3 Kohlenstoffatomen, besonders bevorzugt Methyl oder Ethyl, am bevorzugtesten Methyl.

Alkylreste mit 1 bis 4 Kohlenstoffatomen sind insbesondere ausgewählt aus Methyl, Ethyl, *n*-Propyl, *iso*-Propyl, *n*-Butyl, *sec*-Butyl, *tert*-Butyl, bevorzugt ausgewählt aus Methyl, Ethyl, *n*-Propyl, *iso*-Propyl, *n*-Butyl, *tert*-Butyl, bevorzugter ausgewählt aus Methyl, Ethyl, *iso*-Propyl, tert-Butyl.

Alkylreste mit 1 bis 3 Kohlenstoffatomen sind iausgewählt aus Methyl, Ethyl, *n*-Propyl, *iso*-Propyl, bevorzugt ausgewählt aus Methyl, Ethyl, *iso*-Propyl.

Die Reaktivdestillation zur Herstellung von Alkalimetallalkoholaten ist ein wichtiger industrieller Prozess, da Alkalimetallalkoholate als starke Basen in der Synthese zahlreicher Chemikalien, z.B. bei der Herstellung von Pharma- oder Agrowirkstoffen, und als Katalysatoren in Umesterungs- und Amidierungsreaktionen eingesetzt werden.

Alkalimetallalkoholate ($MOR^{\#}$) werden mittels Reaktivdestillation typischerweise in einer Gegenstromdestillationskolonne aus Alkalimetallhydroxiden (MOH) und Alkoholen ($R^{\#}OH$) hergestellt, wobei das gemäß folgender Reaktion <**1**> entstehende Reaktionswasser mit dem Destillat entfernt wird.

$$MOH + R^{\#} \rightleftharpoons MOR^{\#} + H_2O.$$

Ein solches Verfahrensprinzip ist beispielsweise in der US 2,877,274 A beschrieben, wobei wässrige Alkalimetallhydroxid-Lösung und gasförmiges Methanol im Gegenstrom in einer Rektifikationskolonne gefahren werden. In prinzipiell nicht veränderter Form wird dieses Verfahren in der WO 01/42178 A1 erneut beschrieben.

Die industriell wichtigsten Alkalimetallalkoholate sind jene des Natriums und Kaliums, und hierbei insbesondere die Methylate und Ethylate. Deren Synthese ist vielfach im Stand der Technik beschrieben, zum Beispiel in der EP 1 997 794 A1, der WO 2021/148174 A1 und der WO 2021/148175 A1.

Ähnliche Verfahren, bei denen jedoch zusätzlich noch ein Schleppmittel, wie beispielsweise Benzol, eingesetzt wird, sind in der GB 377,631 A und der US 1,910,331 A beschrieben.

Entsprechend beschreibt die DE 96 89 03 C ein Verfahren zur kontinuierlichen Herstellung von Alkalimetallalkoholaten in einer Reaktionskolonne, wobei das am Kopf entnommene Wasser-Alkohol-Gemisch kondensiert und anschließend einer Phasentrennung unterworfen wird. Die wässrige Phase wird hierbei verworfen und die alkoholische Phase wird zusammen mit dem frischen Alkohol der Kolonne am Kopf zurückgegeben. Ein ähnliches Verfahren beschreibt die EP 0 299 577 A2, wobei die Wasserabtrennung im Kondensat mit Hilfe einer Membran erfolgt.

Anstatt des Alkalimetallhydroxids kann in einer reaktivdestillativen "Umalkoholisierungsreaktion" auch ein anderes Alkalimetallalkoholat MOR* eingesetzt werden und mit dem Alkohol $R^{\#}OH$ zum gewünschten Alkalimetallalkoholat $MOR^{\#}$ und dem Alkohol R*OH umgesetzt werden. R*OH ist dabei üblicherweise ein Alkohol, der niedriger siedet als $R^{\#}OH$. Typischerweise wird Methanol als R*OH bzw. das Methanolat als MOR* eingesetzt.

Solche Umalkoholisierungsreaktionen sind beispielsweise beschrieben in DE 2726491 A1, EP 0776995 oder WO 2021/122702 A1.

In einer bevorzugten Ausführungsform des erfindungsgemäßen Verfahrens wird $\mathbf{S_{AP}}$ in Schritt (a) dadurch erhalten, dass ein Eduktstrom $\mathbf{S_{AE1}}$ umfassend Glykol mit einem Eduktstrom $\mathbf{S_{AE2}}$ umfassend $M_AOR$ im Gegenstrom in einer Reaktivrektifikationskolonne $\mathbf{RR_A}$ zu einem Rohprodukt $\mathbf{RP_A}$ umfassend $M_A$-Glykolat, ROH, Glykol, $M_AOR$ umgesetzt wird, wobei am unteren Ende von $\mathbf{RR_A}$ als Sumpfproduktstrom $\mathbf{S_{AP}}$ entnommen wird.

Noch bevorzugter wird am oberen Ende von $\mathbf{RR_A}$ ein Brüdenstrom $\mathbf{S_{AB}}$ umfassend ROH und gegebenenfalls Glykol entnommen.

Als "Reaktivrektifikationskolonne" wird erfindungsgemäß eine Rektifikationskolonne definiert, in der zumindest in einigen Teilen die Umsetzung gemäß Schritt (a) des erfindungsgemäßen Verfahrens abläuft. Sie kann auch abgekürzt als "Reaktionskolonne" bezeichnet werden.

Gemäß der bevorzugten Ausführungsform des erfindungsgemäßen Verfahrens wird am unteren Ende von $\mathbf{RR_A}$ ein Sumpfproduktstrom $\mathbf{S_{AP}}$ umfassend Glykol und $M_A$-Glykolat entnommen. Am oberen Ende von $\mathbf{RR_A}$ wird ein Brüdenstrom $\mathbf{S_{AB}}$ umfassend ROH und gegebenenfalls Glykol entnommen.

Unter "Glykol" wird im Sinne der Erfindung 1,2-Ethylendiol mit der chemischen Formel $HO\text{-}CH_2\text{-}CH_2\text{-}OH$ (CAS-Nr. 107-21-1) verstanden.

Unter "$M_A$-Glykolat" wird im Sinne der Erfindung das Salz des Glykols mit $M_A$ verstanden. Der Begriff "$M_A$-Glykolat" umfasst mindestens einen von $M_AO\text{-}CH_2\text{-}CH_2\text{-}OH$ und $M_AO\text{-}CH_2\text{-}CH_2\text{-}OM_A$, bevorzugt mindestens $M_AO\text{-}CH_2\text{-}CH_2\text{-}OH$,
am bevorzugtesten $M_AO\text{-}CH_2\text{-}CH_2\text{-}OH$ und $M_AO\text{-}CH_2\text{-}CH_2\text{-}OM_A$.

$M_A$ ist ein Alkalimetall ausgewählt aus Natrium, Kalium und ist bevorzugt Natrium.

Der Eduktstrom $\mathbf{S_{AE1}}$ umfasst Glykol. In einer bevorzugten Ausführungsform liegt der Massenanteil von Glykol in $\mathbf{S_{AE1}}$ bei $\geq$ 95 Gew.-%, noch bevorzugter bei $\geq$ 99.5 Gew.-%, wobei $\mathbf{S_{AE1}}$ ansonsten insbesondere Wasser, Diethylenglykol aufweist.

Das in der bevorzugten Ausführungsform des erfindungsgemäßen Verfahrens als Eduktstrom $\mathbf{S_{AE1}}$ eingesetzte Glykol kann auch handelsübliches Glykol mit einem Glykolmassenanteil von mehr als 99.5 Gew.-% und einem Massenanteil an Wasser von bis zu 0.03 Gew.-%, bis zu 0.05 Gew.-% Diethylenglykol sein.

Der Eduktstrom $\mathbf{S_{AE1}}$ wird in einer Ausführungsform der vorliegenden Erfindung dampfförmig zur Reaktivrektifikationskolonne $\mathbf{RR_A}$ zugegeben.

In einer alternativen, bevorzugten Ausführungsform des erfindungsgemäßen Verfahrens wird Glykol vor Schritt (a) im Sumpf der Reaktivrektifikationskolonne $\mathbf{RR_A}$ vorgelegt, und dann im Schritt (a) zum Sieden erhitzt wird, wodurch in der Reaktivrektifikationskolonne $\mathbf{RR_A}$ ein konstanter Eduktstrom $\mathbf{S_{AE1}}$ erzeugt wird. Gegebenenfalls wird dann während der Durchführung des Schrittes (a) Glykol in den Sumpf der Reaktivrektifikationskolonne $\mathbf{RR_A}$ nachgefüllt.

Der Eduktstrom $\mathbf{S_{AE2}}$ umfasst $M_AOR$. In einer bevorzugten Ausführungsform umfasst $\mathbf{S_{AE2}}$ neben $M_AOR$ mindestens eine weitere Verbindung ausgewählt aus ROH, Glykol. Noch bevorzugter umfasst $\mathbf{S_{AE2}}$ neben $M_AOR$ auch ROH, dann handelt es sich bei $\mathbf{S_{AE2}}$ um eine ROH-alkoholische Lösung von $M_AOR$.

Wenn der Eduktstrom $\mathbf{S_{AE2}}$ $M_AOR$ und ROH umfasst, liegt der Massenanteil von $M_AOR$, bezogen auf das Gesamtgewicht der ROH-alkoholischen Lösung, welche $\mathbf{S_{AE2}}$ bildet, insbesondere im Bereich von 5 bis 75 Gew.-%, bevorzugt von 10 bis 40 Gew.-%, bevorzugter von 15 bis 35 Gew.-%, noch bevorzugter 21 bis 30 Gew.-%.

Für R = Methyl ist der Massenanteil von $M_AOR$, bezogen auf das Gesamtgewicht der ROH-alkoholischen Lösung, welche $\mathbf{S_{AE2}}$ bildet, insbesondere im Bereich von 25 bis 35 Gew.-%, bevorzugt 30 Gew.-%.

Für R = Ethyl ist der Massenanteil von $M_AOR$, bezogen auf das Gesamtgewicht der ROH-alkoholischen Lösung, welche $\mathbf{S_{AE2}}$ bildet, insbesondere im Bereich von 15 bis 25 Gew.-%, bevorzugt 21 Gew.-%.

Der Eduktstrom $\mathbf{S_{AE2}}$ umfasst bevorzugt wenig Wasser, d.h. der Anteil an Wasser im Eduktstrom $\mathbf{S_{AE2}}$ liegt bevorzugt bei < 1 Gew.-%, bevorzugter bei < 0.2 Gew.-%, noch bevorzugter bei < 0.1 Gew.-%, noch bevorzugter bei < 0.01 Gew.-%, jeweils bezogen auf das Gesamtgewicht des Eduktstroms $\mathbf{S_{AE2}}$.

Der Schritt (a) des erfindungsgemäßen Verfahrens wird bevorzugt in einer Reaktivrektifikationskolonne (oder "Reaktionskolonne") $\mathbf{RR_A}$ durchgeführt.

Bevorzugt enthält die Reaktionskolonne $\mathbf{RR_A}$ Einbauten. Geeignete Einbauten sind zum Beispiel Böden, strukturierte Packungen oder unstrukturierte Packungen. Wenn die Reaktionskolonne $\mathbf{RR_A}$ Böden enthält, so sind Glockenböden, Ventilböden, Tunnelböden, Thormann-Böden, Kreuzschlitzglockenböden oder Siebböden geeignet. Wenn die Reaktionskolonne $\mathbf{RR_A}$ Böden enthält, so werden vorzugsweise solche Böden gewählt, bei denen maximal 5 Gew.-%, bevorzugt weniger als 1 Gew.-% der Flüssigkeit durch die jeweiligen Böden durchregnen. Die zur Minimierung des Durchregnens der Flüssigkeit erforderlichen konstruktiven Maßnahmen sind dem Fachmann geläufig. Bei Ventilböden werden zum Beispiel besonders dicht schließende Ventilbauarten gewählt. Durch Verringerung der Zahl der Ventile lässt sich zudem die Dampfgeschwindigkeit in den Bodenöffnungen auf das Doppelte des Wertes, der üblicherweise eingestellt wird, erhöhen. Bei Einsatz von Siebböden ist es besonders günstig, die Durchmesser der Bodenöffnungen zu verringern und die Zahl der Öffnungen beizubehalten oder noch zu vergrößern.

Bei Einsatz von strukturierten oder unstrukturierten Packungen sind strukturierte Packungen im Hinblick auf die gleichmäßige Verteilung der Flüssigkeit bevorzugt.

Der Schritt (a) des erfindungsgemäßen Verfahrens kann sowohl kontinuierlich als auch diskontinuierlich erfolgen. Bevorzugt erfolgt er kontinuierlich.

"Umsetzung eines Eduktstroms $\mathbf{S_{AE1}}$ umfassend Glykol mit einem Eduktstrom $\mathbf{S_{AE2}}$ umfassend $M_AOR$ im Gegenstrom in einer Reaktivrektifikationskolonne $\mathbf{RR_A}$" wird in einer erfindungsgemäßen Ausführungsform insbesondere dadurch gewährleistet, dass die Zulaufstelle mindestens eines Teils des Eduktstroms $\mathbf{S_{AE1}}$ umfassend Glykol an der Reaktionskolonne $\mathbf{RR_A}$ unterhalb der Zulaufstelle des Eduktstroms $\mathbf{S_{AE2}}$ umfassend $M_AOR$ liegt.

In einer alternativen besonderen Ausführungsform kann die "Umsetzung eines Eduktstroms $\mathbf{S_{AE1}}$ umfassend Glykol mit einem Eduktstrom $\mathbf{S_{AE2}}$ umfassend $M_AOR$ im Gegenstrom in einer Reaktivrektifikationskolonne $\mathbf{RR_A}$" auch dadurch gewährleistet werden, dass die Zulaufstelle mindestens eines Teils des Eduktstroms $\mathbf{S_{AE1}}$ umfassend Glykol an der Reaktionskolonne $\mathbf{RR_A}$ oberhalb der Zulaufstelle des Eduktstroms $\mathbf{S_{AE2}}$ umfassend $M_AOR$ liegt.

Die Reaktionskolonne $\mathbf{RR_A}$ umfasst in dieser Ausführungsform vorzugsweise mindestens 2, insbesondere 15 bis 40 theoretische Stufen zwischen der Zulaufstelle des Eduktstroms $\mathbf{S_{AE1}}$ und der Zulaufstelle des Eduktstroms $\mathbf{S_{AE2}}$.

Die Reaktionskolonne $\mathbf{RR_A}$ kann als reine Abtriebskolonne betrieben werden. Dann wird im unteren Bereich der Reaktionskolonne $\mathbf{RR_A}$ dampfförmig der Eduktstrom $\mathbf{S_{AE1}}$ umfassend Glykol zugeführt.

Optional wird ein Teil des Eduktstroms $\mathbf{S_{AE1}}$ umfassend Glykol zwar unterhalb der Zulaufstelle des Eduktstroms $\mathbf{S_{AE2}}$ umfassend $M_AOR$, aber dennoch am oberen Ende oder im Bereich des oberen Endes der Reaktionskolonne $\mathbf{RR_A}$ dampfförmig zugegeben. Dadurch können die Abmessungen im unteren Bereich der Reaktionskolonne $\mathbf{RR_A}$ verringert werden. Wenn ein Teil des Eduktstroms $\mathbf{S_{AE1}}$ umfassend Glykol am oberen Ende oder im Bereich des oberen Endes der Reaktionskolonne $\mathbf{RR_A}$ insbesondere dampfförmig zugegeben wird, so wird bevorzugt nur eine Teilmenge von 10 bis 70 Gew.-%, bevorzugt von 30 bis 50 Gew.-% (jeweils bezogen auf die Gesamtmenge des eingesetzten Glykols) am unteren Ende der Reaktionskolonne $\mathbf{RR_A}$ eingespeist und die restliche Teilmenge in einem Einzelstrom oder auf mehrere Teilströme verteilt, bevorzugt 1 bis 10 theoretische Stufen, besonders bevorzugt 1 bis 3 theoretische Stufen unterhalb der Zulaufstelle des Eduktstroms $\mathbf{S_{AE2}}$ umfassend $M_AOR$ dampfförmig zugegeben.

In einer alternativen Ausführungsform des Schrittes (a) des erfindungsgemäßen Verfahrens wird "Umsetzung eines Eduktstroms $\mathbf{S_{AE1}}$ umfassend Glykol mit einem Eduktstrom $\mathbf{S_{AE2}}$ umfassend $M_AOR$ im Gegenstrom in einer Reaktivrektifikationskolonne $\mathbf{RR_A}$" insbesondere dadurch gewährleistet, dass sich Glykol im Sumpf der Reaktivrektifikationskolonne $\mathbf{RR_A}$ befindet und die Zulaufstelle des Eduktstroms $\mathbf{S_{AE2}}$ umfassend $M_AOR$ oberhalb des Sumpfes liegt. Während des Schrittes (a) des erfindungsgemäßen Verfahrens wird dann Glykol im Sumpf von $\mathbf{RR_A}$ zum Sieden erhitzt und ein Eduktstrom $\mathbf{S_{AE1}}$ umfassend Glykol erzeugt. $\mathbf{S_{AE1}}$ und $\mathbf{S_{AE2}}$ sind dann im Gegenstrom zueinander gerichtet.

In der Reaktionskolonne $\mathbf{RR_A}$ setzt sich dann der Eduktstrom $\mathbf{S_{AE1}}$ umfassend Glykol mit dem Eduktstrom $\mathbf{S_{AE2}}$ umfassend $M_AOR$ zu $M_A$-Glykolat und ROH um, wobei, da es sich um eine Gleichgewichtsreaktion handelt, diese Produkte in Mischung mit den Edukten Glykol und $M_AOR$ vorliegen. Demnach wird in Schritt (a) ein Rohprodukt $\mathbf{RP_A}$ in der Reaktionskolonne $\mathbf{RR_A}$ erhalten, welches neben den Produkten $M_A$-Glykolat und ROH auch noch Glykol und $M_AOR$ umfasst.

Am unteren Ende von $\mathbf{RR_A}$ erhält und entnimmt man dann den Sumpfproduktstrom $S_{AP}$ umfassend Glykol und $M_A$-Glykolat.

Am oberen Ende von $\mathbf{RR_A}$, bevorzugt am Kolonnenkopf von $\mathbf{RR_A}$, entnimmt man in einer bevorzugten Ausführungsform des erfindungsgemäßen Verfahrens, noch gegebenenfalls Glykol enthaltenden Strom von ROH, vorstehend als "Brüdenstrom $\mathbf{S_{AB}}$ umfassend ROH und gegebenenfalls Glykol" bezeichnet.

Enthält der Brüdenstrom $\mathbf{S_{AB}}$ neben ROH auch Glykol, wird, bevorzugt destillativ, zum Beispiel in einer Rektifikationskolonne, Glykol gewonnen. Mindestens ein Teil des bei der Destillation gewonnenen Glykols kann in dieser Ausführungsform der Reaktionskolonne $\mathbf{RR_A}$ als Eduktstrom $\mathbf{S_{AE1}}$ wieder zugeführt werden.

In einer bevorzugten Ausführungsform wird $\mathbf{S_{AB}}$, wenn er neben ROH auch Glykol umfasst, in eine Rektifikationskolonne $\mathbf{RD_A}$ geleitet und in $\mathbf{RD_A}$ in mindestens einen Brüdenstrom $\mathbf{S_{OA}}$ umfassend ROH, der am oberen Ende von $\mathbf{RD_A}$ entnommen wird, und mindestens einen Strom $\mathbf{S_{UA}}$ umfassend Glykol, der am unteren Ende von $\mathbf{RD_A}$ entnommen wird, aufgetrennt.

Die Menge des vom Eduktstrom $\mathbf{S_{AE1}}$ umfassten Glykols wird vorzugsweise so gewählt, dass dieser gleichzeitig als Lösungsmittel für das im Sumpfproduktstrom $\mathbf{S_{AP}}$ erhaltene $M_A$-Glykolat dient. Vorzugsweise wird die Menge des Glykols im Eduktstrom $\mathbf{S_{AE1}}$ so gewählt, dass im Sumpf der Reaktionskolonne die gewünschte Konzentration der $M_A$-Glykolat-Lösung vorliegt, die als Sumpfproduktstrom $\mathbf{S_{AP}}$ umfassend Glykol und $M_A$-Glykolat entnommen wird.

In einer bevorzugten Ausführungsform des erfindungsgemäßen Verfahrens, und insbesondere in den Fällen, in denen $\mathbf{S_{AE2}}$ neben $M_AOR$ auch ROH umfasst, beträgt das Verhältnis des Gesamtgewichts (Massen; Einheit: kg) an als Eduktstrom $\mathbf{S_{AE1}}$ eingesetztem Glykol zum Gesamtgewicht (Massen; Einheit: kg) an als Eduktstrom $\mathbf{S_{AE2}}$ eingesetzten $M_AOR$ 1 : 1 bis 50 : 1, bevorzugter 2 : 1 bis 40 : 1, noch bevorzugter 3 : 1 bis 30 : 1, noch mehr bevorzugter 5 : 1 bis 13.5 :1.

Die Reaktionskolonne $\mathbf{RR_A}$ in der bevorzugten Ausführungsform des erfindungsgemäßen Verfahrens wird mit oder ohne, vorzugsweise mit Rücklauf betrieben.

"Mit Rücklauf' bedeutet, dass der am oberen Ende der jeweiligen Kolonne, insbesondere der Reaktionskolonne $\mathbf{RR_A}$, entnommene Brüdenstrom $\mathbf{S_{AB}}$ umfassend ROH und gegebenenfalls Glykol nicht vollständig abgeführt wird. Der

betreffende Brüdenstrom $\mathbf{S_{AB}}$ wird also mindestens teilweise, bevorzugt teilweise, wieder als Rücklauf der jeweiligen Kolonne, insbesondere der Reaktionskolonne $\mathbf{RR_A}$, zugeführt. In den Fällen, in denen ein solcher Rücklauf eingestellt wird, beträgt das Rücklaufverhältnis dabei bevorzugt 0.01 bis 1, bevorzugter 0.02 bis 0.9, noch bevorzugter 0.03 bis 0.34, besonderes bevorzugt 0.04 bis 0.27 und ganz besonders bevorzugt 0.05 bis 0.24, am bevorzugtesten 0.2.

Unter einem Rücklaufverhältnis wird allgemein und im Sinne dieser Erfindung das Verhältnis aus dem Anteil des aus der Kolonne entnommenen Massenstroms (kg/h), der in flüssiger Form oder gasförmiger Form von der jeweiligen Kolonne abgeführt wird zu dem Anteil dieses Massenstroms (kg/h), der wieder auf die Kolonne in flüssiger Form zurückgeführt wird (Rücklauf), verstanden.

Ein Rücklauf kann dadurch eingestellt werden, dass am Kopf der jeweiligen Kolonne ein Kondensator angebracht wird. Dazu wird insbesondere an der Reaktionskolonne $\mathbf{RR_A}$ ein Kondensator $\mathbf{K_{RRA}}$ angebracht. Im Kondensator $\mathbf{K_{RRA}}$ wird der Brüdenstrom $\mathbf{S_{AB}}$ mindestens teilweise kondensiert und der jeweiligen Kolonne, insbesondere der Reaktionskolonne $\mathbf{RR_A}$ wieder zugeführt.

In der Ausführungsform, in der an der Reaktionskolonne $\mathbf{RR_A}$ ein Rücklauf eingestellt wird, kann der in der bevorzugten Ausführungsform des erfindungsgemäßen Verfahrens als Eduktstrom $\mathbf{S_{AE2}}$ eingesetzte $M_AOR$ auch mindestens teilweise mit dem Rücklaufstrom vermischt werden und die resultierende Mischung so der Reaktionskolonne $\mathbf{RR_A}$ zugeführt werden.

In einer bevorzugten Ausführungsform des erfindungsgemäßen Verfahrens wird Schritt (a) insbesondere bei destillativen Bedingungen durchgeführt, bei denen Glykol refluxiert.

Schritt (a) wird insbesondere bei einer Temperatur im Bereich von 80 °C bis 197 °C, bevorzugt 100 °C bis 197 °C, bevorzugter 120 °C bis 140 °C, und bei einem Druck von 0.01 bar abs. bis 1 bar abs., bevorzugt im Bereich von 0.05 bar abs. bis 1 bar abs., bevorzugter im Bereich von 0.05 bar abs. bis 0.15 bar abs., bevorzugter im Bereich von 0.05 bar abs. bis 0.10 bar abs. durchgeführt.

Die Reaktionskolonne $\mathbf{RR_A}$ umfasst in einer bevorzugteren Ausführungsform mindestens einen Verdampfer, der insbesondere aus Zwischenverdampfern $\mathbf{V_{ZA}}$ und Sumpfverdampfern $\mathbf{V_{SA}}$ ausgewählt ist. Die Reaktionskolonne $\mathbf{RR_A}$ umfasst besonders bevorzugt mindestens einen Sumpfverdampfer $\mathbf{V_{SA}}$.

Als "Zwischenverdampfer" $\mathbf{V_Z}$ werden erfindungsgemäß Verdampfer bezeichnet, die sich oberhalb des Sumpfs der jeweiligen Kolonne, insbesondere oberhalb des Sumpfs der Reaktionskolonne $\mathbf{RR_A}$ (dann als **"**$\mathbf{V_{ZA}}$**"** bezeichnet) bzw. der in der bevorzugten Ausführungsform verwendeten und weiter unten näher beschriebenen Rektifikationskolonne $\mathbf{RD_A}$ (dann als **"**$\mathbf{V_{ZRD}}$**"** bezeichnet) befinden. Im Falle von $\mathbf{RR_A}$ wird in ihnen insbesondere Rohprodukt $\mathbf{RP_A}$ verdampft, das der Kolonne als Seitenstrom $\mathbf{S_{ZAA}}$ entnommen wird.

Als "Sumpfverdampfer" $\mathbf{V_S}$ werden erfindungsgemäß Verdampfer bezeichnet, die den Sumpf der jeweiligen Kolonne, insbesondere den Sumpf der Reaktionskolonne $\mathbf{RR_A}$ bzw. den Sumpf der in der bevorzugten Ausführungsform verwendeten und weiter unten näher beschriebenen Rektifikationskolonne $\mathbf{RD_A}$ (dann als **"**$\mathbf{V_{SRD}}$**"** oder **"**$\mathbf{V_{SRD'}}$**"** bezeichnet) beheizen. Im Falle von $\mathbf{RR_A}$ wird in ihnen insbesondere mindestens ein Teil des Sumpfproduktstroms $\mathbf{S_{AP}}$ verdampft. Im Falle von $\mathbf{RD_A}$ wird in ihnen insbesondere Sumpfproduktstrom $\mathbf{S_{UA}}$ oder ein Teil von $\mathbf{S_{UA}}$, $\mathbf{S_{UA1}}$, verdampft.

Ein Verdampfer ist üblicherweise außerhalb der jeweiligen Reaktionskolonne oder Rektifikationskolonne angeordnet.

Geeignete Verdampfer, die als Zwischenverdampfer und Sumpfverdampfer eingesetzt werden können, sind zum Beispiel Naturumlaufverdampfer, Zwangsumlaufverdampfer, Zwangsumlaufverdampfer mit Entspannung, Kesselverdampfer, Fallfilmverdampfer oder Dünnschichtverdampfer. Als Wärmeübertrager für den Verdampfer wird bei Naturumlaufverdampfern und Zwangsumlaufverdampfern üblicherweise ein Rohrbündel oder Plattenapparat eingesetzt. Bei Einsatz eines Rohrbündelüberträgers kann der Wärmeträger entweder durch die Rohre strömen und das zu verdampfende Gemisch die Rohre umströmen oder aber der Wärmeträger umströmt die Rohre und das zu verdampfende Gemisch durchströmt die Rohre. Bei einem Fallfilmverdampfer wird das zu verdampfende Gemisch üblicherweise als dünner Film auf der Innenseite eines Rohres zugegeben und das Rohr wird von außen beheizt. Im Unterschied zu einem Fallfilmverdampfer ist in einem Dünnschichtverdampfer zusätzlich ein Rotor mit Wischern vorgesehen, der die zu verdampfende Flüssigkeit auf der Innenwand des Rohres zu einem dünnen Film verteilt.

Neben den genannten kann aber auch jede beliebige andere, dem Fachmann bekannte Verdampferbauart, die sich zum Einsatz an einer Rektifikationskolonne eignet, eingesetzt werden.

In der bevorzugten Ausführungsform des erfindungsgemäßen Verfahrens wird am unteren Ende der Reaktionskolonne $\mathbf{RR_A}$ $\mathbf{S_{AP}}$ umfassend Glykol und $M_A$-Glykolat als Sumpfproduktstrom entnommen.

Es ist bevorzugt, dass die Reaktionskolonne $\mathbf{RR_A}$ mindestens einen Sumpfverdampfer $\mathbf{V_{SA}}$ aufweist, über den Sumpfproduktstrom $\mathbf{S_{AP}}$ dann teilweise geleitet wird und Glykol aus diesem teilweise entfernt wird, wodurch ein Sumpfproduktstrom $\mathbf{S_{AP*}}$ mit einem gegenüber $\mathbf{S_{AP}}$ erhöhten Massenanteil an $M_A$-Glykolat erhalten wird.

Insbesondere weist im erfindungsgemäßen Verfahren $\mathbf{S_{AP}}$ oder, falls mindestens einen Sumpfverdampfer $\mathbf{V_{SA}}$ eingesetzt wird, über den der Sumpfproduktstrom $\mathbf{S_{AP}}$ mindestens teilweise geleitet wird und Glykol aus diesem mindestens teilweise entfernt wird, $\mathbf{S_{AP*}}$, einen Massenanteil von $M_A$-Glykolat in Glykol im Bereich von 1 bis 50 Gew.-%, bevorzugt 5 bis 35 Gew.-%, bevorzugter 15 bis 35 Gew.-%, am bevorzugtesten 20 bis 35 Gew.-% auf, jeweils bezogen

auf die Gesamtmasse von $\mathbf{S_{AP}}$.

Der Massenanteil an ROH in $\mathbf{S_{AP}}$ bzw. $\mathbf{S_{AP*}}$ liegt dabei bevorzugt bei < 1 Gew.-%, bevorzugt < 0.8 Gew.-%, bevorzugter < 0.5 Gew.-%, bezogen auf die Gesamtmasse von $\mathbf{S_{AP}}$.

Der Massenanteil an Wasser in $\mathbf{S_{AP}}$ bzw. $\mathbf{S_{AP*}}$ liegt dabei bevorzugt bei < 1 Gew.-%, bevorzugt < 0.8 Gew.-%, bevorzugter < 0.5 Gew.-%, bezogen auf die Gesamtmasse von $\mathbf{S_{AP}}$.

Der Massenanteil an Edukt $M_AOR$ in $\mathbf{S_{AP}}$ bzw. $\mathbf{S_{AP*}}$ liegt dabei bevorzugt bei < 1 Gew.-%, bevorzugt < 0.8 Gew.-%, bevorzugter < 0.5 Gew.-%, bevorzugter < 0.005 Gew.-%, bezogen auf die Gesamtmasse von $\mathbf{S_{AP}}$.

Der Massenanteil an Wasser in $\mathbf{S_{AE1}}$ liegt dabei bevorzugt bei < 1 Gew.-%, bevorzugt < 0.8 Gew.-%, bevorzugter < 0.1 Gew.-%, bevorzugter < 0.005 Gew.-%, bezogen auf die Gesamtmasse von $\mathbf{S_{AE1}}$.

Der Massenanteil an Wasser in $\mathbf{S_{AE2}}$ liegt dabei bevorzugt bei < 1 Gew.-%, bevorzugt < 0.8 Gew.-%, bevorzugter < 0.1 Gew.-%, bevorzugter < 0.005 Gew.-%, bezogen auf die Gesamtmasse von SAE2.

In einer noch bevorzugteren Ausführungsform des erfindungsgemäßen Verfahrens wird am oberen Ende von $\mathbf{RR_A}$ ein Brüdenstrom $\mathbf{S_{AB}}$ umfassend ROH und gegebenenfalls Glykol entnommen.

2. Rektifikation des Brüdenstroms $\mathbf{S_{AB}}$ in einer Rektifikationskolonne $\mathbf{R_{DA}}$ (bevorzugt)

Der Brüdenstrom $\mathbf{S_{AB}}$ wird, wenn er ROH und Glykol umfasst, in einer weiteren bevorzugten Ausführungsform in eine Rektifikationskolonne $\mathbf{RD_A}$ geleitet und in $\mathbf{RD_A}$ in mindestens einen Brüdenstrom $\mathbf{S_{OA}}$ umfassend ROH, der am oberen Ende von $\mathbf{RD_A}$ entnommen wird, und mindestens einen Strom $\mathbf{S_{UA}}$ umfassend Glykol, der am unteren Ende von $\mathbf{RD_A}$ entnommen wird, aufgetrennt.

"Mindestens ein Brüdenstrom $\mathbf{S_{OA}}$ umfassend ROH, der am oberen Ende von $\mathbf{RD_A}$ entnommen wird" bedeutet, dass der Brüden, der am oberen Ende von $\mathbf{RD_A}$ erhalten wird, dort als ein oder mehrere Brüdenströme entnommen werden kann.

"Mindestens einen Strom $\mathbf{S_{UA}}$ umfassend Glykol, der am unteren Ende von $\mathbf{RD_A}$ entnommen wird" bedeutet, dass Glykol, das am unteren Ende von $\mathbf{RD_A}$ erhalten wird, dort als ein oder mehrere Ströme entnommen werden kann.

Der Brüdenstrom $\mathbf{S_{AB}}$ kann dabei über eine oder mehrere Zulaufstellen in die Rektifikationskolonne $\mathbf{RD_A}$ geleitet werden. In den Ausführungsformen der vorliegenden Erfindung, in denen der Brüdenstrom $\mathbf{S_{AB}}$ als zwei oder mehrere voneinander getrennte Ströme in die Rektifikationskolonne $\mathbf{R_{DA}}$ geleitet wird, ist es vorteilhaft, wenn die Zulaufstellen der einzelnen Ströme im Wesentlichen auf der gleichen Höhe an der Rektifikationskolonne $\mathbf{RD_A}$ liegen.

In einer bevorzugten Ausführungsform des erfindungsgemäßen Verfahrens wird der Brüdenstrom $S_{AB}$, wenn er ROH und Glykol umfasst, in einer Rektifikationskolonne $\mathbf{RD_A}$ in einen Brüdenstrom $\mathbf{S_{OA}}$ umfassend ROH, der am oberen Ende von $\mathbf{RD_A}$ entnommen wird, und einen Strom $\mathbf{S_{UA}}$ umfassend Glykol, der am unteren Ende von $\mathbf{RD_A}$ entnommen wird, aufgetrennt.

Eine andere Bezeichnung für "oberes Ende einer Rektifikationskolonne" ist "Kopf".

Eine andere Bezeichnung für "unteres Ende einer Rektifikationskolonne" ist "Sumpf" oder "Fuß".

Als Rektifikationskolonne $\mathbf{RD_A}$ kann jede beliebige, dem Fachmann bekannte Rektifikationskolonne eingesetzt werden.

Bevorzugt enthält die Rektifikationskolonne $\mathbf{RD_A}$ Einbauten. Geeignete Einbauten sind zum Beispiel Böden, unstrukturierte Packungen oder strukturierte Packungen. Als Böden werden üblicherweise Glockenböden, Siebböden, Ventilböden, Tunnelböden oder Schlitzböden eingesetzt. Unstrukturierte Packungen sind im Allgemeinen Füllkörperschüttungen. Als Füllkörper werden üblicherweise Raschigringe, Pallringe, Berl-Sättel oder Intalox®-Sättel verwendet. Strukturierte Packungen werden zum Beispiel unter dem Handelsnamen Mellapack® der Firma Sulzer vertrieben. Neben den genannten Einbauten weitere geeignete Einbauten sind dem Fachmann bekannt und können ebenfalls verwendet werden.

Bevorzugte Einbauten weisen einen geringen spezifischen Druckverlust pro theoretischer Trennstufe auf. Strukturierte Packungen und Füllkörper haben zum Beispiel einen deutlich kleineren Druckverlust pro theoretischer Trennstufe als Böden. Dies hat den Vorteil, dass der Druckverlust in der Rektifikationskolonne $\mathbf{RD_A}$ möglichst gering bleibt und somit die mechanische Leistung des Verdichters und die Temperatur des zu verdampfenden Glykol/ROH-Gemischs gering bleibt.

Wenn in der Rektifikationskolonne $\mathbf{RD_A}$ strukturierte Packungen oder unstrukturierte Packungen enthalten sind, so können diese geteilt sein oder es kann eine durchgehende Packung vorliegen. Üblicherweise sind jedoch mindestens zwei Packungen vorgesehen, eine Packung oberhalb der Zulaufstelle des Brüdenstroms $\mathbf{S_{AB}}$ und eine Packung unterhalb der Zulaufstelle des Brüdenstroms $\mathbf{S_{AB}}$. Es kann auch eine Packung oberhalb der Zulaufstelle des Brüdenstroms $\mathbf{S_{AB}}$ und mehrere Böden unterhalb der Zulaufstelle des Brüdenstroms $\mathbf{S_{AB}}$ vorgesehen sein. Wenn eine unstrukturierte Packung eingesetzt wird, beispielsweise eine Füllkörperpackung, so liegen die Füllkörper üblicherweise auf einem geeigneten Tragrost (z.B. Siebboden oder Gitterboden) auf.

In dieser bevorzugten Ausführungsform wird dann der mindestens eine Brüdenstrom $\mathbf{S_{OA}}$ umfassend ROH am oberen Ende der Rektifikationskolonne $\mathbf{RD_A}$ entnommen. Der bevorzugte Massenanteil von ROH in diesem Brüdenstrom

$\mathbf{S_{OA}}$ ist ≥ 99 Gew.-%, bevorzugter ≥ 99.6 Gew.-%, noch bevorzugter ≥ 99.9 Gew.-%, wobei der Rest insbesondere Glykol ist.

Am unteren Ende von $\mathbf{RD_A}$ wird in dieser bevorzugten Ausführungsform mindestens ein Strom $\mathbf{S_{UA}}$ umfassend Glykol entnommen, der bevorzugt < 1 Gew.-%, bevorzugter ≤ 5000 Gew.-ppm, noch bevorzugter ≤ 1000 Gew.-ppm ROH, bevorzugter ≤ 100 Gew.-ppm ROH aufweisen kann.

Die Entnahme mindestens einen Brüdenstroms $\mathbf{S_{OA}}$ umfassend ROH am Kopf der Rektifikationskolonne $\mathbf{RD_A}$ bedeutet im Rahmen der vorliegenden Erfindung insbesondere, dass der mindestens eine Brüdenstrom $\mathbf{S_{OA}}$ als Kopfstrom oder als Seitenabzug oberhalb der Einbauten in der Rektifikationskolonne $\mathbf{RD_A}$ entnommen wird.

Die Entnahme des mindestens einen Stroms $\mathbf{S_{UA}}$ umfassend Glykol am Sumpf der Rektifikationskolonne $\mathbf{RD_A}$ bedeutet im Rahmen der vorliegenden Erfindung insbesondere, dass der mindestens ein Strom $\mathbf{S_{UA}}$ als Sumpfstrom oder am unteren Boden der Rektifikationskolonne $\mathbf{RD_A}$ entnommen wird.

Die Rektifikationskolonne $\mathbf{RD_A}$ wird mit oder ohne, vorzugsweise mit Rücklauf betrieben.

"Mit Rücklauf" bedeutet, dass der am oberen Ende der Rektifikationskolonne $\mathbf{RD_A}$ entnommene Brüdenstrom $\mathbf{S_{OA}}$ nicht vollständig abgeführt wird, sondern teilweise kondensiert und wieder der jeweiligen Rektifikationskolonne $\mathbf{RD_A}$ zugeführt wird. In den Fällen, in denen ein solcher Rücklauf eingestellt wird, beträgt das Rücklaufverhältnis dabei bevorzugt 0.01 bis 1, bevorzugter 0.02 bis 0.9, noch bevorzugter 0.03 bis 0.34, besonderes bevorzugt 0.04 bis 0.27 und ganz besonders bevorzugt 0.05 bis 0.24, am bevorzugtesten 0.2.

Ein Rücklauf kann dadurch eingestellt werden, dass am Kopf der Rektifikationskolonne $\mathbf{RD_A}$ ein Kondensator $\mathbf{K_{RD}}$ angebracht wird. In dem Kondensator $\mathbf{K_{RD}}$ wird der jeweilige Brüdenstrom $\mathbf{S_{OA}}$ teilweise kondensiert und der Rektifikationskolonne $\mathbf{RD_A}$ wieder zugeführt.

## 3. Schritt (b): Umsetzung von PET mit der Lösung $\mathbf{S_{AP}}$

In Schritt (b) des erfindungsgemäßen Verfahrens wird die in Schritt (a) erhaltene Lösung $\mathbf{S_{AP}}$ umfassend Glykol und $M_A$-Glykolat mit **PET** zu einer Mischung $\mathbf{M_1}$ umfassend **BHET** umgesetzt.

### *3.1 PET-Ausgangsmaterial*

Als **PET,** welches im Schritt (b) des erfindungsgemäßen Verfahrens eingesetzt wird, kann jegliches **PET** eingesetzt werden, was depolymerisiert werden muss. Typischerweise fällt solches **PET** als Abfall an, insbesondere im Haushalt, in der Industrie oder in der Landwirtschaft.

In einer Ausführungsform des erfindungsgemäßen Verfahrens liegt das damit zu depolymerisierende **PET** in Mischung mit anderen Kunststoffen, insbesondere mindestens einem Kunststoff ausgewählt aus Polyethylen ("PE"), Polyvinylchlorid ("PVC") vor. Dies ist typischerweise der Fall, wenn im erfindungsgemäßen Verfahrens **PET** aus Kunststoffabfällen depolymerisiert werden soll. In dieser Ausführungsform wird das **PET** mindestens teilweise von den anderen Kunststoffen, bevorzugt durch Aussortieren, abgetrennt, bevor es dem Schritt (b) des erfindungsgemäßen Verfahrens unterworfen wird.

In einer Ausführungsform des erfindungsgemäßen Verfahrens wird das **PET** mindestens einem Vorbehandlungsschritt ausgesetzt.

Solche Vorbehandlungsschritte sind beispielsweise beschrieben in DE 10032899 C2.

Erfindungsgemäß wird das **PET** mindestens einem Vorbehandlungsschritt ausgewählt aus chemischen Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugt mindestens einem Vorbehandlungsschritt ausgewählt aus mindestens teilweiser Abtrennung von anderen Kunststoffen, bevorzugt durch Aussortierung, chemischen Vorbehandlungsschritt, Zerkleinerungsschritt unterworfen, bevor es in Schritt (b) eingesetzt wird.

In den Fällen, in denen das **PET** in Mischung mit anderen Kunststoffen vorliegt, wird das **PET** bevorzugter erst mindestens teilweise von anderen Kunststoffen abgetrennt, dann mindestens einmal chemisch vorbehandelt und schließlich zerkleinert.

Bei dem chemischen Vorbehandlungsschritt handelt es sich insbesondere um einen Waschschritt. Ein solcher Waschschritt hat den Vorteil, dass vor der Durchführung des Schrittes (b) eventuelle Verunreinigungen, insbesondere Speisereste, Reste von Kosmetika und/oder Körpersekrete (z.B. Blut, Sperma, Fäkalien), entfernt werden. Solche Verunreinigungen könnten die Effizienz der Umsetzung in Schritt (b) herabsetzen und/oder die Reinheit des damit erhaltenen **BHET** verschlechtern.

Beim chemischen Vorbehandlungsschritt, insbesondere dem Waschschritt, wird der Abfall insbesondere in einer Waschlösung bei einer Temperatur von 30 °C bis 99 °C, bevorzugt 50 C° bis 90 °C, noch bevorzugter 70 °C bis 85 °C, erhitzt.

Typische Waschlösungen sind dem Fachmann geläufig und bevorzugt ausgewählt aus:

- wässrige Lösung eines Tensids, bevorzugt eines nicht-ionischen Tensids;
- wässrige Lösung eines Alkalimetallhydroxids oder Erdalkalimetallhydroxids; bevorzugt wässrige NaOH.

Die Behandlungszeit des chemischen Vorbehandlungsschritts, insbesondere des Waschschritts, beträgt dabei insbesondere 1 min bis 12 h, bevorzugt 10 min bis 6 h, bevorzugter 30 min bis 2 h, noch bevorzugter 45 bis 90 min, am bevorzugtesten 60 min.

Nach der Behandlung des **PET** mit dem chemischen Vorbehandlungsschritt, insbesondere dem Waschschritt, wird die wässrige Lösung abgetrennt, z.B. durch Filtration, und das gesäuberte **PET** bevorzugt mindestens einmal mit Wasser gewaschen, um Reste der Waschlösung zu entfernen.

Der so erhaltene PET-Abfall wird dann getrocknet, insbesondere in einem Trockenschrank.

Die zum Trocknen eingesetzt Temperatur liegt dabei insbesondere im Bereich 30 bis 120 °C, bevorzugt 50 °C bis 100 °C, bevorzugter 60 °C bis 90 °C, am bevorzugtesten bei 80 °C.

Der Zerkleinerungsschritt hat den Vorteil, dass die in Schritt (b) für die Reaktion zur Verfügung stehende Oberfläche des **PET** erhöht wird. Dadurch erhöht sich die Reaktionsgeschwindigkeit der Umsetzung in Schritt (b) erhöht. Die Zerkleinerung kann in den Fachmann bekannten Apparaturen erfolgen, beispielsweise einem Shredder oder einer Schneidmühle.

In einer weiteren Ausführungsform des erfindungsgemäßen Verfahrens wird das **PET,** bevor es dem Schritt (b) unterworfen wird, entfärbt oder gezielt gefärbt. Dies kann mit dem Fachmann bekannten Verfahren durchgeführt werden, z.B. Entfärbung mit Wasserstoffperoxid oder Färbung mit einem Farbstoff.

*3.2 Umsetzungsbedingungen*

Die Umsetzung des **PET** mit einer Lösung $\mathbf{S_{AP}}$ umfassend Glykol und $M_A$-Glykolat zu einer Mischung $\mathbf{M_1}$ kann dann unter dem Fachmann geläufigen Bedingungen erfolgen.

Bevorzugt wird die Umsetzung in Schritt (b) so lange, d.h. bis zu einem Zeitpunkt $\mathbf{t_b}$, durchgeführt bis sich mindestens **P** = 10 %, bevorzugt mindestens **P** = 20 %, bevorzugter mindestens **P** = 25 %, bevorzugter mindestens **P** = 30 %, bevorzugter mindestens **P** = 40 %, bevorzugter mindestens **P** = 50 %, bevorzugter mindestens **P** = 60 %, bevorzugter mindestens **P** = 70 %, bevorzugter mindestens **P** = 80 %, bevorzugter mindestens P = 90 %, bevorzugter mindestens **P** = 95 %, noch bevorzugter mindestens **P** = 99 % des in Schritt (b) eingesetzten **PET**s umgesetzt haben.

Dieser prozentuale Anteil **P** wird nach der folgenden Formel berechnet:

$$\mathbf{P} = (n_{\mathbf{TS}} + n_{\mathbf{MHET}} + n_{\mathbf{BHET}}) / n_{\mathbf{PET}}.$$

Dabei ist $n_{PET}$ die Stoffmenge an Wiederholungseinheiten der folgenden Struktur (Ξ) in dem in Schritt (b) eingesetzten **PET:**

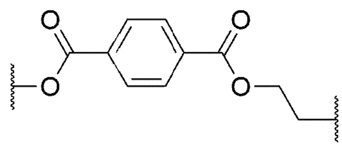

**(Ξ)**

$n_{TS}$ ist die Stoffmenge an **TS,** die sich von Beginn des Schrittes (b) bis zum Zeitpunkt $\mathbf{t_b}$ in Schritt (b) gebildet haben.

$n_{MHET}$ ist die Stoffmenge an **MHET,** die sich von Beginn des Schrittes (b) bis zum Zeitpunkt $\mathbf{t_b}$ in Schritt (b) gebildet haben.

$n_{BHET}$ ist die Stoffmenge an **BHET,** die sich von Beginn des Schrittes (b) bis zum Zeitpunkt $\mathbf{t_b}$ in Schritt (b) gebildet haben.

Die Strukturen der Verbindungen **BHET, MHET, TS** sind wie folgt:

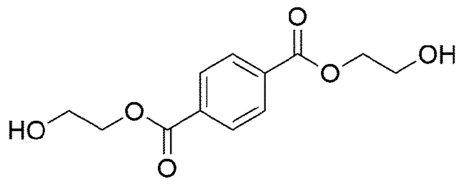

**BHET**

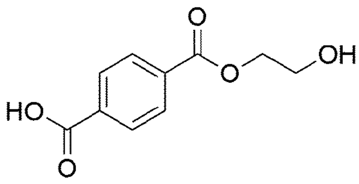

**MHET**

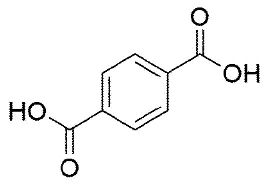

**TS**

**"MHET"** umfasst auch das entsprechende Carboxylat der gezeigten Struktur. **"TS"** umfasst auch das entsprechende Mono- und Dicarboxylat der gezeigten Struktur.

Die Umsetzung in Schritt (b) wird dabei insbesondere bei einer Temperatur von mindestens 100 °C, bevorzugt bei einer Temperatur im Bereich von ≥ 100 °C bis ≤ 197 °C, bevorzugter bei einer Temperatur im Bereich von ≥ 130 °C bis ≤ 197 °C, bevorzugter bei einer Temperatur im Bereich von ≥ 150 °C bis ≤ 197 °C, bevorzugter bei einer Temperatur im Bereich von ≥ 175 °C bis ≤ 197 °C durchgeführt.

Die Umsetzung in Schritt (b) wird bevorzugt bei der Siedetemperatur des Glykols, durchgeführt. Noch bevorzugter wird Glykol dabei refluxiert, das heißt Glykol wird aus der Umsetzung verdampft, kondensiert und dann wieder in die Umsetzung rückgeführt. Diese Refluxierung kann mit dem Fachmann geläufigen Mitteln eingestellt werden, beispielsweise in einer Destillationsapparatur.

Das Gesamtgewicht des im Verfahren eingesetzten $M_A$-Glykolats bezogen auf das Gesamtgewicht des im Verfahren eingesetzten **PET**s liegt insbesondere im Bereich von 0.1 bis 100 Gew.-%, bevorzugt im Bereich von 0.5 bis 80 Gew.-%, bevorzugter im Bereich von 1.0 bis 50 Gew.-%, bevorzugter im Bereich von 1.5 bis 25 Gew.-%, bevorzugter im Bereich von 2.0 bis 10 Gew.-%, bevorzugter im Bereich von 2.5 bis 6.0 Gew.-%, besonders bevorzugt bei 3.5 bis 5.0 Gew-%, am bevorzugtesten bei 3.9 Gew.-%.

Die Umsetzung in Schritt (b) kann mit dem Fachmann geläufigen Geräten erfolgen.

Nach Beendigung des Schrittes (b) des erfindungsgemäßen Verfahrens wird eine Mischung $M_1$ erhalten, in der das molare Verhältnis η der Stoffmenge von **BHET** ($n_{BHET}$) zur Summe der Stoffmengen von **MHET** und **TS** ($n_{MHET} + n_{TS}$) im Bereich 1 : 1 bis 1000 : 1, bevorzugt 2 : 1 bis 500 : 100, bevorzugter 4 : 1 bis 300 : 1, noch bevorzugter 10 : 1 bis 100 : 1, noch mehr bevorzugter 13 : 1 bis 60 : 1, noch mehr bevorzugter 13 : 1 bis 24 : 1 liegt.

$$\eta = n_{BHET} / (n_{MHET} + n_{TS})$$

*3.3 Bevorzugter Schritt (c)*

In einem bevorzugten, weiteren Schritt (c) wird **BHET** mindestens teilweise aus $M_1$ abgetrennt. Dies erfolgt noch bevorzugter durch Kristallisation und/oder Destillation. Noch bevorzugter wird **BHET** in Schritt (c) aus $\mathbf{M_1}$ abfiltriert und dann auskristallisiert.

## 4. Verfahren zum Recycling von **PET**

Das im erfindungsgemäßen Verfahren in der Mischung $M_1$ erhaltene **BHET** wird bevorzugt in einem Verfahren zum Recycling von Polyethylenterephthalat in einem Schritt (ζ) zu **PET** polymerisiert.

Diese Polymerisierung ist dem Fachmann als "Polykondensation" bekannt und z.B. in EP 0 723 951 A1 und von Th. Rieckmann und S. Völker in Kapitel 2 "Poly(Ethylen Terephthalate) Polymerization - Mechanism, Catalysis, Kinetics, Mass Transfer and Reactor Design" auf Seite 92 des Buches " Modern Polyesters: Chemistry and Technology of Polyesters and Copolyesters. Edited by J. Scheirs and T. E. Long, 2003, John Wiley & Sons, Ltd ISBN: 0-471-49856-4" beschrieben.

Insbesondere wird dazu **BHET** im Schritt (ζ) in Gegenwart von Katalysatoren, bei denen es sich insbesondere um Katalysatoren, die aus der Gruppe bestehend aus Antimonverbindungen, bevorzugt $Sb_2O_3$, ausgewählt sind, handelt, wieder zu **PET** polymerisiert.

Bevorzugt wird die Polymerisierung von **BHET** zu **PET** in Schritt (ζ) mindestens bei der Siedetemperatur des Glykols durchgeführt. Insbesondere wird während der Polymerisierung in Schritt (ζ) Glykol aus dem Reaktionsgemisch entfernt, um das Reaktionsgleichgewicht auf die Seite des Polymers **PET** zu verschieben.

Bevorzugter wird die Polymerisierung von **BHET** zu **PET** in Schritt (ζ) bei der Siedetemperatur des Glykols durchgeführt. Noch bevorzugter wird dann während der Polymerisierung in Schritt (ζ) Glykol aus dem Reaktionsgemisch entfernt, um das Reaktionsgleichgewicht auf die Seite des Polymers **PET** zu verschieben.

Dies wird insbesondere durch Destillation bei einem Druck < 1 bar, bevorzugt 0.1 mbar bei der gleichzeitigen Siedetemperatur des Glykols beim jeweiligen Druck erreicht.

## Beispiele

1. Erfinderisches Beispiel **E1:**

*1.1 Herstellung der glykolischen Natriumglykolatösung durch Reaktivdestillation*

Die folgende Apparatur wird als Destillationsapparatur genutzt:
Als Vorlagegefäß bzw. Sumpf der Destillationsapparatur dient ein beheizbares 2.5 l - Doppelmantelgefäß mit Temperaturfühler und vakuumdichtem Rührer. Darüber befindet sich eine 25 cm-Kolonne mit Multifill-Packung und Silberspiegel (Abtriebsteil). Die Zudosierung von Natriummethanolat ("NaOMe") erfolgt oberhalb der Kolonne mithilfe eines Tropftrichters. Oberhalb der Zudosierung befindet sich eine weitere Kolonne, die der Abtrennung von Ethylenglykol und Methanoldampf dient (Verstärkerteil). Mithilfe eines Dampfteilers im oberen Teil der Kolonne kann ein Rücklaufverhältnis eingestellt werden, wobei das Destillat in einem Rundkolben gesammelt wird. Der Rundkolben kann über einen Tropftrichter mit Druckausgleich vom Destillationssystem getrennt und ausgewechselt werden. Am Verstärkerteil ist ein Rückflusskühler mit Vakuumanschlüssen angeschlossen, über den die gesamte Apparatur evakuiert werden kann. Das Vakuum wird über eine Drehschieberpumpe erzeugt, welche mit zwei Kühlfallen und einer Sicherheitsflasche an die Destillationsapparatur angeschlossen wird. Der Druck in der Destillationsapparatur wird an der Sicherheitsflasche (Büchi-Vakuumcontroller) gemessen, wo auch eine Belüftung erfolgen kann. Die Sumpfvorlage, sowie die Kolonne mit der Multifill Packung sind zur Isolierung komplett mit Aluminiumfolie umgeben, um eine gleichbleibende Temperatur im Reaktor/ in der Kolonne zu gewährleisten.

Im Sumpf wird das Ethylenglykol vorgelegt und die gesamte Apparatur wird auf 50 mbar evakuiert. Anschließend wird der Sumpf bis zur Siedetemperatur erhitzt, sodass sich ein Rücklauf aus dem Verstärkerteil einstellt. Anschließend wir NaOMe (30 Gew.-% in Methanol) mithilfe eines Tropftrichters zudosiert. Die Dosierrate wird so gewählt, dass die methanolische NaOMe-Lösung nicht den Sumpf erreicht (ca. 2 mL/min).

Das hinzugefügte bzw. entstehende Methanol wird im Verstärkerteil destillativ von Ethylenglykol getrennt und im Rundkolben aufgefangen. Das Rücklaufverhältnis beträgt 5 : 1 (5 Teile als Rücklauf, 1 Teil als Destillat). Die abdestillierte Menge muss dabei mindestens der hinzugefügten Menge Methanol entsprechen. Nach dem Abdestillieren wird das Natriumethylenglykolat im Sumpf noch ca. 2 Stunden nachdestilliert (bei gleichbleibendem Vakuum und Temperatur wird das im Verstärkerteil vorhandene Methanol entfernt, um ein Rückfließen in den Sumpf zu verhindern).

Nach Beendigung und Abkühlen des Versuches wird der Sumpf über ein Ablassventil geöffnet und ca. 20 Gew.-% ige Lösung von Natriumglykolat in Ethylenglykol abgenommen.

*1.2 Depolymerisierung **PET** mit glykolischer Natriumglykolatösung aus der Reaktivdestillation*

Im erfindungsgemäßen Verfahren werden 100 g **PET** in einem Autoklaven mit 800 g Ethylenglykol vorgelegt. Die Lösung wird anschließend unter Rühren auf 150 °C erhitzt. Sobald die Temperatur von 150 °C erreicht ist, werden 19.5 g 20%-ige Natriumglykolat-Lösung in Ethylenglykol (entsprechend 0.046 mol) aus der Reaktivdestillation hinzugefügt. Die Reaktion wird über fünf Stunden durchgeführt und der Reaktoraustrag nach dem Abkühlen untersucht. Der erhaltene Umsatz an **BHET** (1) und 2-Hydroxyethylterephthalsäure (= **"MHET")** (2) sowie Terephthalsäure (= "**TS**") (3) wird per Gaschromatographie bestimmt.

2. Vergleichsbeispiel **V1:**

In einem Vergleichsversuch werden 100 g **PET** in einem Autoklaven mit 800 g Ethylenglykol vorgelegt. Die Lösung wird anschließend unter Rühren auf 150 °C erhitzt. Die Reaktion wird über fünf Stunden durchgeführt und der Reaktoraustrag nach dem Abkühlen untersucht. Der erhaltene Umsatz an **BHET** (1) und **MHET** (2) sowie **TS** (3) wird per Gaschromatographie bestimmt.

3. Vergleichsbeispiel **V2:**

In einem Vergleichsversuch werden 100 g **PET** in einem Autoklaven mit 800 g Ethylenglykol vorgelegt. Die Lösung wird anschließend unter Rühren auf 150 °C erhitzt. Sobald die Temperatur von 150 °C erreicht ist, werden 3.7 g 50 Gew.-%-ige NaOH-Lösung in Wasser (entsprechend 0.046 mol) hinzugefügt. Die Reaktion wird über fünf Stunden durchgeführt und der Reaktoraustrag nach dem Abkühlen untersucht. Der erhaltene Umsatz an **BHET** (1) und **MHET** (2) sowie **TS** (3) wird per Gaschromatographie bestimmt.

4. Ergebnis

Der Vergleich des Gehalts an **BHET, MHET** und **TS** im depolymerisierten Produkt im erfinderischen Beispiel **E1** und den Vergleichsbeispielen **V1, V2** zeigt, dass bei der Depolymerisierung unter Einsatz der durch Reaktivdestillation erhaltenen glykolischen Natriumglykolatlösung ein höherer Anteil an **BHET** erhalten wird. Dies ist vorteilhaft, da dadurch mehr Produkt zur Verfügung steht, welches direkt in einer Polykondensation zu neuem Produkt **PET** umgesetzt werden kann.

## Patentansprüche

1. Verfahren zur Depolymerisierung von Polyethylenterephthalat **PET,** umfassend die folgenden Schritte:

   (a) $M_A$OR und Glykol werden in einer Reaktivdestillation umgesetzt, wodurch eine Lösung $\mathbf{S_{AP}}$ umfassend Glykol und $M_A$-Glykolat erhalten wird, wobei $M_A$ ein Alkalimetall ausgewählt aus Natrium, Kalium ist, und wobei R ein Alkylrest mit 1 bis 4 Kohlenstoffatomen ist,
   (b) Umsetzung der Lösung $\mathbf{S_{AP}}$ mit **PET** zu einer Mischung $M_1$ umfassend Bis-2-hydroxyethylterephthalat **BHET.**

2. Verfahren nach Anspruch 1, wobei $\mathbf{S_{AP}}$ im Schritt (a) dadurch erhalten wird, dass ein Eduktstrom $\mathbf{S_{AE1}}$ umfassend Glykol mit einem Eduktstrom $S_{AE2}$ umfassend $M_A$OR im Gegenstrom in einer Reaktivrektifikationskolonne $\mathbf{RR_A}$ zu einem Rohprodukt $\mathbf{RP_A}$ umfassend $M_A$-Glykolat, ROH, Glykol, $M_A$OR umgesetzt wird,
   wobei $\mathbf{S_{AP}}$ am unteren Ende von $\mathbf{RR_A}$ als Sumpfproduktstrom entnommen wird.

3. Verfahren nach Anspruch 2, wobei am oberen Ende von $\mathbf{RR_A}$ ein Brüdenstrom $\mathbf{S_{AB}}$ umfassend ROH und gegebenenfalls Glykol entnommen wird.

4. Verfahren nach Anspruch 3, wobei $\mathbf{S_{AB}}$ ROH und Glykol umfasst, in eine Rektifikationskolonne $\mathbf{RD_A}$ geleitet wird und in $\mathbf{RD_A}$ in mindestens einen Brüdenstrom $\mathbf{S_{OA}}$ umfassend ROH, der am oberen Ende von $\mathbf{RD_A}$ entnommen wird, und mindestens einen Strom $\mathbf{S_{UA}}$ umfassend Glykol, der am unteren Ende von $\mathbf{RD_A}$ entnommen wird, aufgetrennt wird.

5. Verfahren nach einem der Ansprüche 1 bis 4, wobei Schritt (b) so lange durchgeführt wird, bis sich mindestens **P** = 10 % des in Schritt (b) eingesetzten **PET**s umgesetzt haben.

6. Verfahren nach einem der Ansprüche 1 bis 5, wobei der Gehalt von ROH in $\mathbf{S_{AP}}$ bei < 1 Gew.-% liegt.

7. Verfahren nach einem der Ansprüche 1 bis 6, wobei Schritt (b) bei der Siedetemperatur des Glykols durchgeführt wird.

8. Verfahren nach einem der Ansprüche 1 bis 7, wobei in Schritt (b) so viel $\mathbf{S_{AP}}$ eingesetzt wird, dass das Gesamtgewicht des in Schritt (b) eingesetzten $M_A$-Glykolats bezogen auf das Gesamtgewicht des in Schritt (b) eingesetzten **PET**s im Bereich von 0.1 bis 100 Gew.-% liegt.

9. Verfahren nach einem der Ansprüche 1 bis 8, wobei **BHET** in einem weiteren Schritt (c) mindestens teilweise aus $M_1$ abgetrennt wird.

10. Verfahren nach Anspruch 9, wobei die mindestens teilweise Abtrennung von **BHET** aus $M_1$ in Schritt (c) durch Kristallisation und/oder Destillation erfolgt.

11. Verfahren nach einem der Ansprüche 1 bis 10, wobei das **PET** mindestens einem Vorbehandlungsschritt ausgewählt aus chemischen Vorbehandlungsschritt, Zerkleinerungsschritt unterworfen wird, bevor es in Schritt (b) eingesetzt wird.

12. Verfahren zum Recycling von Polyethylenterephthalat **PET,** in welchem mit einem Verfahren nach einem der Ansprüche 1 bis 11 **BHET** erhalten wird und in einem Schritt ($\zeta$) das so erhaltene **BHET** zu **PET** polymerisiert wird.

13. Verfahren nach Anspruch 12, wobei die Polymerisierung von **BHET** zu **PET** in Schritt ($\zeta$) bei mindestens der Siedetemperatur des Glykols durchgeführt wird.

14. Verfahren nach Anspruch 12 oder 13, wobei die Polymerisierung in Schritt ($\zeta$) in Gegenwart eines Katalysators

durchgeführt wird.

**15.** Verfahren nach Anspruch 14, wobei der Katalysator aus der Gruppe bestehend aus Antimonverbindungen ausgewählt ist.

**Claims**

**1.** Method of depolymerization of polyethylene terephthalate PET, comprising the following steps:

(a) converting $M_AOR$ and glycol in a reactive distillation to obtain a solution $S_{AP}$ comprising glycol and $M_A$ glycolate, where $M_A$ is an alkali metal selected from sodium, potassium, and where R is an alkyl radical having 1 to 4 carbon atoms,
(b) reacting the solution $S_{AP}$ with PET to give a mixture $M_1$ comprising bis-2-hydroxyethyl terephthalate BHET.

**2.** Method according to Claim 1, wherein $S_{AP}$ is obtained in step (a) by reacting a reactant stream $S_{AE1}$ comprising glycol with a reactant stream $S_{AE2}$ comprising $M_AOR$ in countercurrent in a reactive rectification column $RR_A$ to give a crude product $RP_A$ comprising $M_A$ glycolate, ROH, glycol, $M_AOR$,
wherein $S_{AP}$ is withdrawn as bottom product stream at the lower end of $RR_A$.

**3.** Method according to Claim 2, wherein a vapour stream $S_{AB}$ comprising ROH, with or without glycol, is withdrawn at the upper end of $RR_A$.

**4.** Method according to Claim 3, wherein $S_{AB}$ comprises ROH and glycol, is directed into a rectification column $RD_A$ and is separated in $RD_A$ into at least one vapour stream $S_{OA}$ comprising ROH which is withdrawn at the upper end of $RD_A$, and at least one stream $S_{UA}$ comprising glycol which is withdrawn at the lower end of $RD_A$.

**5.** Method according to any of Claims 1 to 4, wherein step (b) is conducted until at least P = 10% of the PET used in step (b) has been converted.

**6.** Method according to any of Claims 1 to 5, wherein the content of ROH in $S_{AP}$ is < 1% by weight.

**7.** Method according to any of Claims 1 to 6, wherein step (b) is performed at the boiling temperature of the glycol.

**8.** Method according to any of Claims 1 to 7, wherein a sufficient amount of $S_{AP}$ is used in step (b) that the total weight of the $M_A$ glycolate used in step (b), based on the total weight of the PET used in step (b), is in the range from 0.1% to 100% by weight.

**9.** Method according to any of Claims 1 to 8, wherein BHET is at least partly separated from $M_1$ in a further step (c).

**10.** Method according to Claim 9, wherein the at least partial separation of BHET from $M_1$ in step (c) is effected by crystallization and/or distillation.

**11.** Method according to any of Claims 1 to 10, wherein the PET is subjected to at least one pretreatment step selected from chemical pretreatment step, comminution step, before being used in step (b).

**12.** Method of recycling polyethylene terephthalate PET, in which BHET is obtained by a method according to any of Claims 1 to 11 and the BHET thus obtained is polymerized to PET in a step ($\zeta$).

**13.** Method according to Claim 12, wherein the polymerization of BHET to PET in step ($\zeta$) is conducted at at least the boiling temperature of the glycol.

**14.** Method according to Claim 12 or 13, wherein the polymerization in step ($\zeta$) is performed in the presence of a catalyst.

**15.** Method according to Claim 14, wherein the catalyst is selected from the group consisting of antimony compounds.

## Revendications

1. Procédé de dépolymérisation de poly(téréphtalate d'éthylène) PET, comprenant les étapes suivantes :

   (a) transformation de $M_AOR$ et de glycol dans une distillation réactive, suite à quoi une solution $S_{AP}$ comprenant du glycol et du glycolate de $M_A$ est obtenue, $M_A$ représentant un métal alcalin choisi parmi le sodium et le potassium et R représentant un radical alkyle comprenant 1 à 4 atomes de carbone,
   (b) transformation de la solution $S_{AP}$ avec du PET en un mélange $M_1$ comprenant du téréphtalate de bis-2-hydroxyéthyle BHET.

2. Procédé selon la revendication 1, dans lequel la solution $S_{AP}$ dans l'étape (a) est obtenue en ce qu'un flux de départ $S_{AE1}$ comprenant du glycol est transformé avec un flux de départ $S_{AE2}$ comprenant du $M_AOR$ à contre-courant dans une colonne de rectification réactive $RR_A$ en un produit brut $RP_A$ comprenant du glycolate de $M_A$, du ROH, du glycol, du $M_AOR$,
   la solution $S_{AP}$ étant prélevée au niveau de l'extrémité inférieure de $RR_A$ en tant que flux de fond produit.

3. Procédé selon la revendication 2, dans lequel un flux de vapeur $S_{AB}$ comprenant du ROH et le cas échéant du glycol est prélevé au niveau de l'extrémité supérieure de $RR_A$.

4. Procédé selon la revendication 3, dans lequel la solution $S_{AB}$ comprend du ROH et du glycol, est guidée dans une colonne de rectification $RD_A$ et est séparée dans $RD_A$ en au moins un flux de vapeur $S_{OA}$ comprenant du ROH, qui est prélevé au niveau de l'extrémité supérieure de $RD_A$, et en au moins un flux $S_{UA}$ comprenant du glycol, qui est prélevé au niveau de l'extrémité inférieure de $RD_A$.

5. Procédé selon l'une des revendications 1 à 4, dans lequel l'étape (b) est réalisée jusqu'à ce qu'au moins P = 10% du PET utilisé dans l'étape (b) soient transformés.

6. Procédé selon l'une des revendications 1 à 5, dans lequel la teneur en ROH dans la solution $S_{AP}$ est < 1% en poids.

7. Procédé selon l'une des revendications 1 à 6, dans lequel l'étape (b) est réalisée à la température d'ébullition du glycol.

8. Procédé selon l'une des revendications 1 à 7, dans lequel, dans l'étape (b), on utilise une quantité de $S_{AP}$ telle que le poids total du glycolate de $M_A$ utilisé dans l'étape (b), par rapport au poids total du PET utilisé dans l'étape (b), se situe dans la plage de 0,1 à 100% en poids.

9. Procédé selon l'une des revendications 1 à 8, dans lequel le BHET est séparé au moins partiellement à partir de $M_1$ dans une autre étape (c).

10. Procédé selon la revendication 9, dans lequel la séparation au moins partielle du BHET à partir de $M_1$ dans l'étape (c) est effectuée par cristallisation et/ou distillation.

11. Procédé selon l'une des revendications 1 à 10, dans lequel le PET est soumis à au moins une étape de prétraitement choisie parmi une étape de prétraitement chimique et une étape de broyage avant d'être utilisé dans l'étape (b).

12. Procédé de recyclage de poly(téréphtalate d'éthylène) PET, dans lequel, à l'aide d'un procédé selon l'une des revendications 1 à 11, du BHET est obtenu et, dans une étape ($\zeta$), le BHET ainsi obtenu est polymérisé en PET.

13. Procédé selon la revendication 12, dans lequel la polymérisation de BHET en PET dans l'étape ($\zeta$) est réalisée à au moins la température d'ébullition du glycol.

14. Procédé selon la revendication 12 ou 13, dans lequel la polymérisation dans l'étape ($\zeta$) est réalisée en présence d'un catalyseur.

15. Procédé selon la revendication 14, dans lequel le catalyseur est choisi dans le groupe constitué par les composés d'antimoine.

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

### In der Beschreibung aufgeführte Patentdokumente


- GB 784248 A
- JP 2000309663 A
- US 4355175 A
- EP 0723951 A1
- US 3222299 A
- WO 2020002999 A2
- EP 1457479 A1
- US 2877274 A
- WO 0142178 A1
- EP 1997794 A1
- WO 2021148174 A1
- WO 2021148175 A1
- GB 377631 A
- US 1910331 A
- DE 968903 C
- EP 0299577 A2
- DE 2726491 A1
- EP 0776995 A
- WO 2021122702 A1
- DE 10032899 C2


### In der Beschreibung aufgeführte Nicht-Patentliteratur


- *CHEMICAL ABSTRACTS*, 959-26-2
- **W. CASERI**. *Polyethylenterephthalate*, 2009
- **F. BÖCKLER** ; **B. DILL** ; **G. EISENBRAND** ; **F. FAUPEL** ; **B. FUGMANN** ; **T. GAMSE** ; **R. MATISSEK** ; **G. POHNERT** ; **A. RÜHLING** ; **S. SCHMIDT**. RÖMPP [Online. Georg Thieme Verlag, January 2022
- **T. YOSHIOKA** ; **N. OKAYAMA** ; **A. OKUWAKI**. *Ind. Eng. Chem. Res.*, 1998, vol. 37, 336-340
- **S.R. SHUKLA** ; **A.M. HARAD**. *Journal of Applied Polymer Science*, 2005, vol. 97, 513-517
- **N.D. PINGALE** ; **S.R. SHUKLA**. *European Polymer Journal*, 2008, vol. 44, 4151-4156
- *CHEMICAL ABSTRACTS*, 107-21-1
- Poly(Ethylen Terephthalate) Polymerization - Mechanism, Catalysis, Kinetics, Mass Transfer and Reactor Design. **TH. RIECKMANN** ; **S. VÖLKER**. Modern Polyesters: Chemistry and Technology of Polyesters and Copolyesters.. John Wiley & Sons, Ltd, 2003, 92